# EUROPEAN PATENT SPECIFICATION

(11) **EP 4 473 919 B1**
(45) Date of publication and mention of the grant of the patent: **11.02.2026**
(21) Application number: 24180423.6
(22) Date of filing: 06.06.2024
(51) Int. Cl.: A61B 17/132, A61B 17/00

(54) **PHLEBOLOGICAL DEVICE**
PHLEBOLOGISCHE VORRICHTUNG
DISPOSITIF PHLÉBOLOGIQUE

(30) Priority: 07.06.2023 IT 202300011667
(43) Date of publication of application: 11.12.2024
(73) Proprietor: Ceschin, Maurizio, 34015 Muggia (TS) (IT)
(72) Inventor: Ceschin, Maurizio, 34015 Muggia (TS) (IT)
(74) Representative: Giugni, Diego

(56) References cited:
- CN-U- 215 273 103
- US-A- 4 224 945
- US-A1- 2014 163 606

## Description

### TECHNICAL FIELD OF THE INVENTION

The present invention relates to a phlebological device for the treatment of blood vessels. In particular, the invention relates to a particularly versatile phlebological device such to be capable of standardising the application of eccentric pressure in a controlled and adjustable manner depending on the specificity involved, all this with simple and precise operations.

### BACKGROUND OF THE INVENTION

Phlebology is a branch of medicine that deals with the diagnosis and treatment of venous diseases. Among these, the most common are varices (varicose veins), reticular ectasias and capillary ectasias (telangiectasias) which may affect both upper and lower limbs. Phlebology also deals with the prevention of complications of these diseases, including phlebothrombosis or thrombophlebitis events (same disease but caused by different factors) and varicose ulcers of the lower limbs that manifest themselves with lesions, pain, bleeding and swelling at the ankles and which often require hospitalization.

In particular, in the treatment of varicose veins, various endovascular techniques, comprising thermal exploitation techniques, for irradiation, chemical and hybrid techniques, have been developed. In general, regardless of the type of technique, patients need to wear containment clothing, such as elastic stockings, or laborious bandages, often combined with eccentric compression elements (*eccentric bodies )* made by hand. As a matter of fact, it is known that after procedures such as sclerotherapy, there arises the need to apply a so-called eccentric pressure at the treated vessel that acts only in the section involved in the procedure and it remains stable there for as long as is necessary to achieve the desired result and the correct effectiveness of the treatment.

Currently, the compression elements used by physicians can take many different forms precisely because there are no standard devices on the market that can adapt to any situation, that is, that is they are not versatile. Therefore, physicians, in the truest sense of the word, prepare haphazard compression elements for example formed by rolls of gauzes, pads, cotton masses, cardboard tubes, rolled drapes and other solutions that also partly depend on the creativity of the operator. These elements are placed along the path or skin area below which the treatment was carried out and blocked there with bandages or containment stockings.

Although such solutions are considered to be the common practice up to now, none can guarantee standardization of application and corresponding versatility to adapt to all types of cases dealt with.

In addition, the application requires a certain skilled dexterity both to correctly position unstable and uneven shaped elements as well as to hold them in place after bandaging or application of a containment stocking. As a matter of fact, it has been observed that very frequently both when applying and with the movement of the person, the elements tend to move from the target position, thus affecting their correct functionality.

US 4 224945 describes a device which has the purpose of providing means which exert tension on the portion of intact skin surrounding an ulcer in order to promote its healing. Furthermore, the device provides means for exerting a compressive force on the surface of the ulcer to promote inward growth of new skin and to counteract the tendency for new skin to grow at the edge of the ulcer.

US 2014/163606 describes an adhesive bandage sandwich for compressing blood vessels, comprising a first adhesive bandage layer suitable for adhering to human skin, substantially transparent, an elastic body of stable shape with the ability to change shape and a second adhesive bandage layer suitable for adhering to the first adhesive layer.

CN 215 272 103 U describes a very complex haemostasis device at the puncture site of an arteriovenous fistula. The device compresses the internal fistula blood vessels in a targeted manner, reminds the duration of different compression states in real time and displays the pressure of the puncture point in real time by means of sensors for detecting and controlling compression chambers.

### SUMMARY OF THE INVENTION

Therefore, the object of the present invention is to provide a phlebological device that solves the problems outlined above.

Therefore, a first object of the invention is a standardised phlebological device, that is which can be applied under any post-operative condition.

A second object is a phlebological device that does not require complex operations for the application thereof.

A further object is a phlebological device that is stable and safe over time so as to ensure its action over the entire required duration thereof.

A yet further object is a phlebological device that can be adjusted depending on the progress of the healing or on the needs of the patient at any time without having to restore the entire activity for applying or replacing the entire device.

In addition, a different object is a kit for assembling the device of the invention.

### BRIEF DESCRIPTION OF THE FIGURES

Advantages, characteristics and other objects of the invention will be apparent from the following description, provided by way of non-limiting example, with reference to the attached figures, wherein:
- figure 1 shows a perspective view of a phlebological device of the invention from its skin application side;
- figure 2 shows a plan schematic view of a portion of an aligned chain of devices of figure 1 connected along their longitudinal axis X-X;
- figure 3 shows a lateral cross-sectional view along the longitudinal axis X-X of the device of figure 2;
- figure 4 shows a perspective view of a cross-section along line III-III of the device of figure 1;
- figure 5 shows a first step for preparing the device of figure 1 ;
- figure 6 shows a step for applying the device of figure 1 on a portion of a lower limb of a patient;
- figures 7A and 7B respectively show a cross-sectional view of the limb portion at a blood vessel on which the device of figure 1 is applied, and a cross-sectional view of the application of a containment bandage on said device.

### DETAILED DESCRIPTION OF THE INVENTION

The idea underlying the present invention is to standardise a method for the treatment of varices and capillaries so as to facilitate and enhance the precision of the task of the medical staff, under any pathological condition, with only one device. To this end, it was decided to develop a device comprising at least one tubular element adapted to be pressurized and provided with at least one portion of the outer surface coated by an adhesive layer for contact with the skin of a patient so that it can be precisely applied on the treated area and remain thereon. Suitably repeatable, this element is then kept active by compression means.

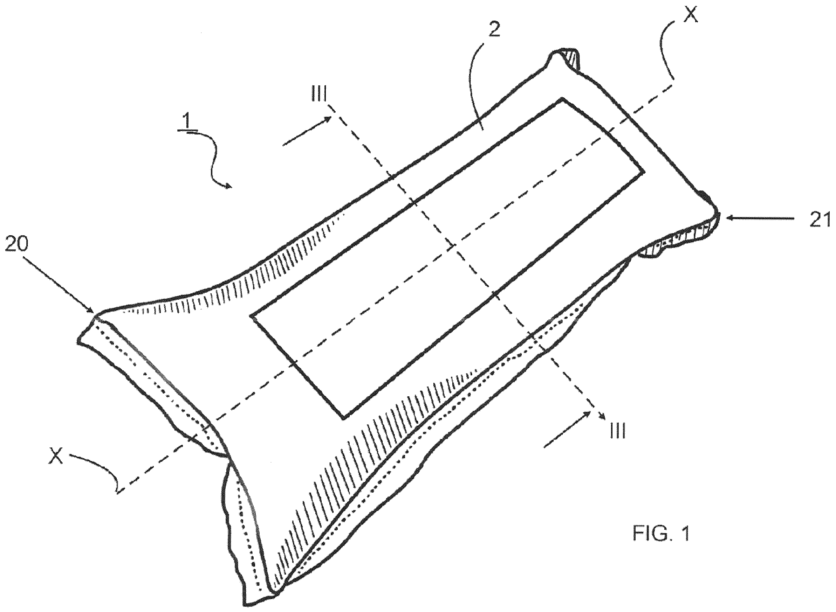

In particular, the device of the invention is indicated with reference numeral 1 in figure 1. It comprises at least one functional tubular or sac element 2 made of inflatable plastic adapted to transfer the pressure exerted from the external on a well-defined area of a limb of a patient (see for example figures 1, 3 and 4).

The tubular element 2 is a sort of inflatable sac extending along a longitudinal axis X-X from a first 20 to a second end 21. Both ends are sealed or can be sealed hermetically so that it can contain a pressurised fluid or gas and, therefore, act as a compressible cushion to (evenly) transfer pressure at the area where it is applied. Preferably, the element is filled with an inert gas, for example air, with a pressure generally such to fully expand the internal space and cause the full tension of the walls of the element. In any case, the amount of fluid or gas and the pressure can be adjusted according to particular needs or preferences, for example taking into account the body mass overlying the area to be treated.

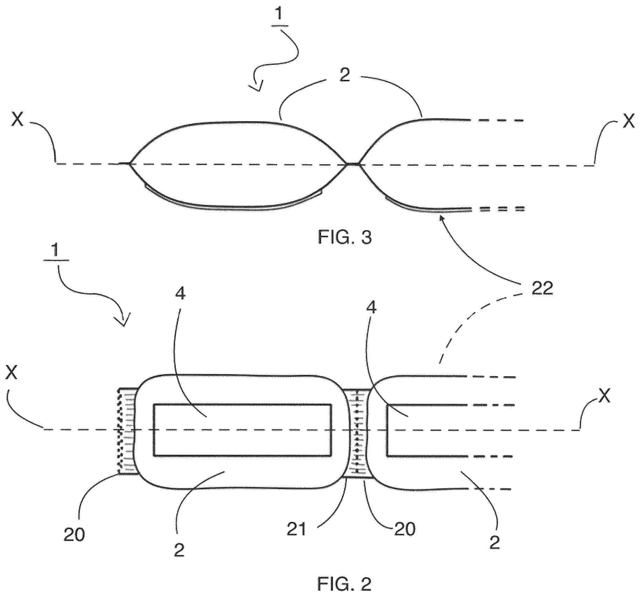

According to an embodiment, the device of the invention comprises a plurality of tubular elements 2 joined together at the respective first 20 and second 21 end so as to form a sort of chain 22, as shown in the figures 2 and 3. This chain may derive from an individual long tubular element which is filled with a fluid to obtain the inflation thereof and subsequently transversely split in individual separate elements with desired length by welding the inner surfaces. Otherwise, individual inflated tubular elements 2 may be joined together using glue, connecting means such as Velcro, snap fasteners, buttons and corresponding holes, hooks and corresponding holes or the like, or simply arranged adjacent to each other.

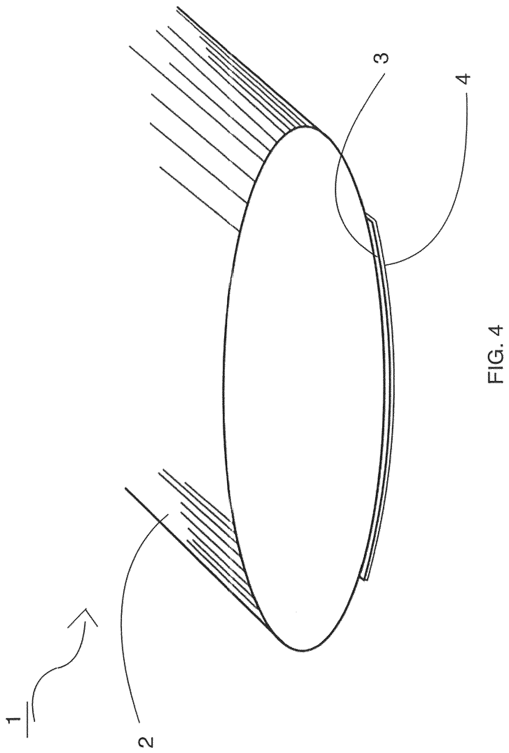

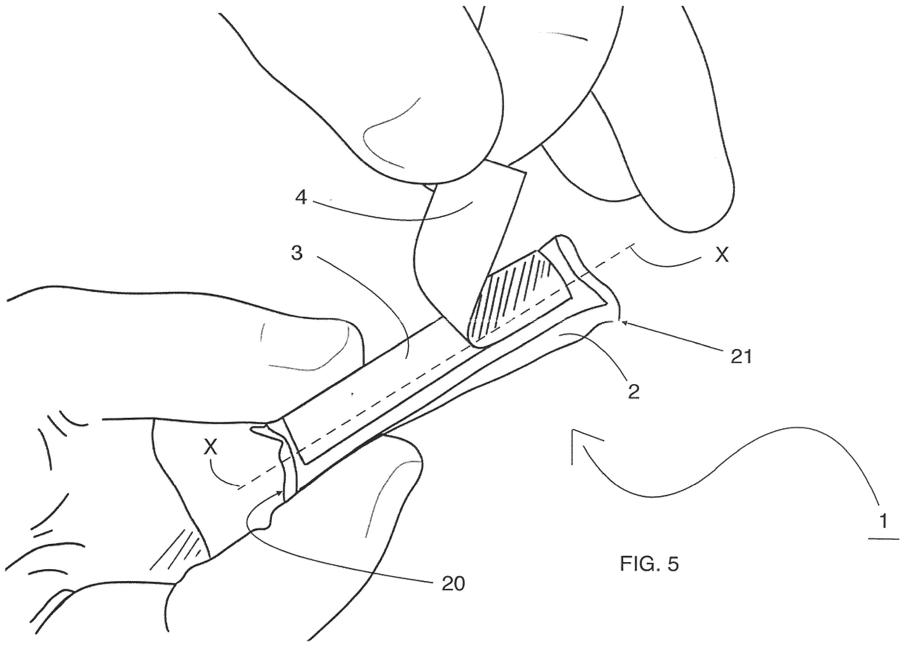

Advantageously, the tubular element 2 mentioned above comprises a portion of its outer surface coated by a layer of glue 3 (figures 4 and 5), preferably biocompatible and anergic. This layer of glue 3 is in turn covered by a protective film 4 made of paper, plasticised paper or plastic, which can be removed to apply the element on the limb of a patient. The layer of glue 3 preferably extends along the entire axis X-X of the element 2 from the first 20 to the second 21 end on its central portion. Alternatively, the glue may involve up to half of the circumferential surface of the tubular element, or it may be distributed with transversal strips.

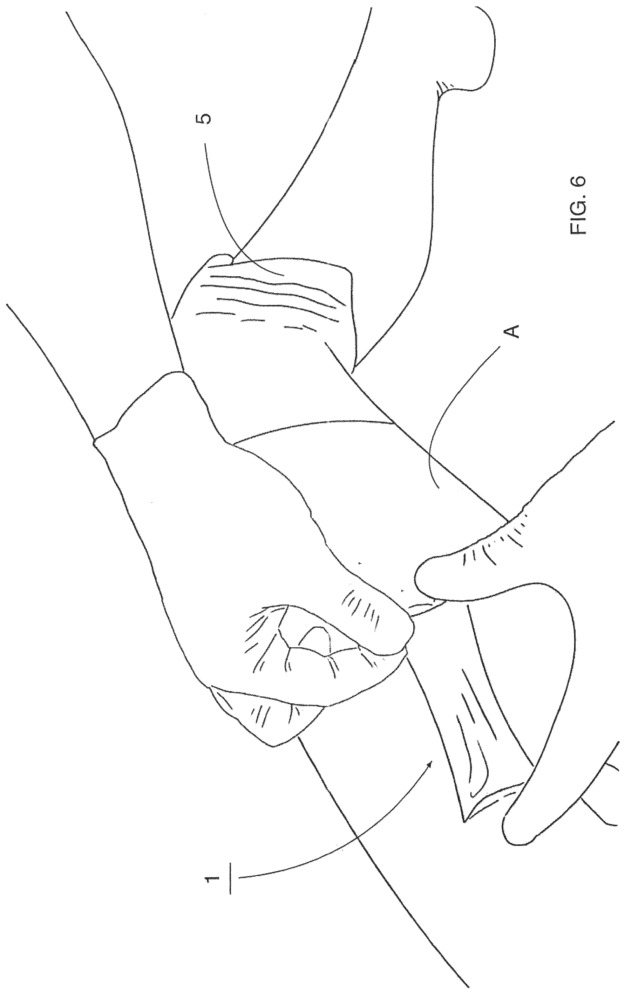

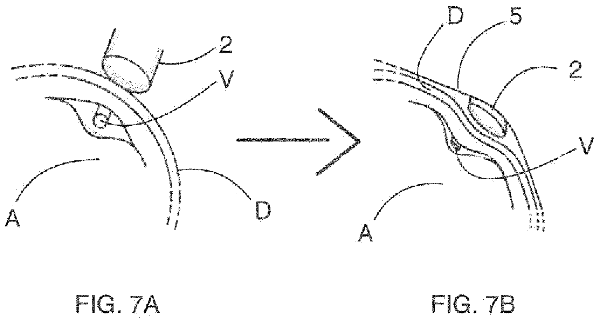

The phlebological device 1 also comprises compression means 5 (figures 6 and 7) for said tubular element 2 which can be applied on its outer surface opposite to the surface coated by the adhesive layer 3. These means may be a bandage or gauze which is manually wrapped with various layers by a single strip of fabric exerting the pressure required to block the terminal end of the strip with conventional stops such as adhesive tapes or elastic hooks. Alternatively, said means may be a conventional strip or elastic stocking to be worn around the limb portion on which the tubular element 2 mentioned above is applied being superimposed thereto to exert the required pressure.

In the light of the above, it can be easily deduced that the phlebological device of the invention allows an applicability that is simple, quick, highly efficient and highly versatile given that it is standardised. As a matter of fact, as shown in the figures 5-7, the application of the device 1 provides for a few steps which essentially comprise the positioning of one or more tubular elements 2 at the point for applying pressure on the limb A of a patient, as shown in figure 6 representing a portion of a lower limb of a patient, after removing the protective film 4 of the layer of glue 3 as shown in figure 5. In particular, as shown by the enlargement of figures 7A and 7B, a tubular element 2 suitably inflated according to the recommendations of the physician depending on the conditions of the patient, is adhered to the dermis D for example of a section of the venous vessel V, such as the saphenous vein. Thanks to the adhesive layer, the tubular element 2 remains in position advantageously allowing the operator to handle a compression means, such as an elastic stocking or a bandage, without any concern whatsoever on an easy shifting of the tubular element. Being thus free, the operator may proceed without further aid with the excellent result for applying a pre-established and controlled/controllable pressure due to the characteristics of the inflated tubular element 2 mentioned above (figure 7B).

Therefore, the vein portion affected by the pressure will be effectively crushed specifically due to the transfer of the pressure exerted by the compression means 5 by the tubular element 2.

In the light of the above, it is clear that the drawbacks related to systems and devices of the prior art outlined above have been overcome. In particular, the invention allows to avoid hand-made pressure transfer elements that are not easy to control, repeatable with the same characteristics, therefore which can be standardised. Furthermore, the invention allowed to eliminate the potential unwanted shifting from the required point of application.

By contrast, the phlebological device of the invention is perfectly controllable, it can be made up of multiple elements with characteristics which can be standardised, on the one hand, and which can be adjusted depending on the specific needs, on the other hand. As a matter of fact, should the device comprise a plurality of tubular elements extending in sections of a limb of a patient with different anatomical characteristics (greater thickness of the adipose tissue under the dermis, thicker muscle layer, etc.), the different tubular elements may be inflated at different pressure values for an almost perfect adaptation to the anatomy of the person.

In this regard, it should be observed that according to a preferred embodiment of the invention, the tubular elements 2 may be provided with valves (not shown) for the filling thereof as needed and according to the physician's recommendations. Such valves may be conventional check valves, that is that allow the inflow of air and block the outflow thereof, unless the walls of the air inflow hole are suitably squeezed.

Therefore, according to a further object of the present invention, the phlebological device 1 comprises an assembly kit provided with a single tubular element 2 made of inflatable plastic like the one described above and which extends for a length of a few metres and which can be cut depending on the needs, compression means 5, inflation means (not shown), and an apparatus (not shown) for the transversal sealing of sections of said element. The element may be provided with the valves mentioned above distributed at pre-determined distances along the longitudinal axis thereof and, obviously, on the wall opposite to the one carrying the adhesive layer 3 mentioned above. The sealing apparatus may be a conventional commercial equipment such as for example one provided with a plate that can be heated which melts the two transversal opposite strips of the tube of the element 2 through heat. The inflation means may be manual or electrical air pumps of the commercial type.

The phlebological device of the present invention may be subjected - by the person skilled in the art - to numerous modifications and variants but without departing from the scope of protection of the attached claims.

For example, the materials used for the tubular element may be conventional anergic plastics such as polyethylene. The glues of the adhesive layer may be biocompatible glue, also anergic.

The dimensions of the tubular element may vary depending on the specific needs or preferences, same case applying to those of the compression means. In particular, the phlebological device represents an eccentric body, once the compression means are applied around the limb of the patient, given that it is only there that there is a different pressure value considering the circumference of the limb on which the compression means are wound.

Furthermore, the individual tubular element may be produced with any desired size and, preferably, for the treatment of telangiectasias and reticular veins the dimensions in the swollen conditions are 15×70 mm and for the collateral saphenous veins and the great and small saphenous veins measure 35×90 mm.

## Claims

1. Phlebological device (1) comprising at least one tubular element (2) adapted to be pressurized and further comprising compression means (5) of said tubular element applicable on the outer surface of the device, **characterized in that** said tubular element (2) is provided with at least one outer portion coated by an adhesive layer (3) for contact with the skin of a patient, said adhesive layer being covered by a removable protective strip (4), and further said compression means (5) for said element are applicable onto the outer surface of the device, opposed to said surface coated by the adhesive layer, and they are a bandage, gauze, compression-elastic band or stockings to be worn around the limb portion on which said tubular element is applied.

2. Phlebological device (1) according to claim 1, wherein said tubular element (2) is made of sealable inflatable anergic plastic and it extends along a rectilinear axis (X-X) between a first (29) and a second (21) sealable end for containing a pressurized fluid.

3. Phlebological device (1) according to claim 1 or 2, wherein said tubular element (2) is formed by a plurality of single tubular elements (2) connected each other at said first (20) and second end (21) so as to form a chain (22).

4. Phlebological device (1) according to any one of claims 1 to 3, wherein said tubular elements (2) are connected by means of connecting means comprising glue for joining two opposite inner strips of the tube, welding of said two opposite inner strips of the tube, or Velcro, snap fasteners, buttons and corresponding holes, hooks and corresponding holes.

5. Phlebological device (1) according to any one of claims 2 to 4, wherein said adhesive layer (3) is a strip of biocompatible and anergic glue extending along said axis (X-X) of the tubular element (2) from its first (20) to the second (21) end and on its central portion.

6. Phlebological device (1) according to any one of claims 2 to 4, wherein said adhesive layer (3) is at least one strip of biocompatible and anergic glue extending transversal to said axis (X-X) up to half of its surface.

7. Phlebological device (1) according to any one of claims 1 to 6, wherein said tubular element (2) comprises at least one check valve for its controlled and adjustable inflation.

8. Phlebological kit comprising at least one tubular element (2) according to claim 1 made of inflatable plastic extending along a longitudinal axis (X-X), compression means (5) of said tubular element when it is inflated, means for inflating said tubular element, and an apparatus for sealing said tubular element.

9. Phlebological kit according to claim 8, wherein said inflation means are a manual or electric air pump, said sealing apparatus comprises a plate that can be heated for fusing two opposite strips of the tubular element (2).

## Patentansprüche

1. Phlebologische Vorrichtung (1), die mindestens ein röhrenförmiges druckbeaufschlagbares Element (2) und außerdem eine Kompressionseinrichtung (5) für das röhrenförmige Element aufweist, die auf die Außenfläche der Vorrichtung anbringbar ist,
**dadurch gekennzeichnet, dass**
das röhrenförmige Element (2) mit mindestens einem Außenbereich versehen ist, der mit einer Haftschicht (3) zum Kontakt mit der Haut eines Patienten beschichtet ist, wobei die Haftschicht mit einem abnehmbaren Schutzstreifen (4) bedeckt ist, und dass die Kompressionseinrichtung (5) für das Element auf die Außenfläche der Vorrichtung aufbringbar ist, die der mit der Haftschicht beschichteten Fläche gegenüberliegt, und dass es sich dabei um einen Verband, eine Gaze, ein kompressionselastisches Band oder Strümpfe handelt, die um den Gliedmaßenabschnitt getragen werden, auf den das röhrenförmige Element aufgebracht wird.

2. Phlebologische Vorrichtung (1) nach Anspruch 1, wobei das röhrenförmige Element (2) aus abdichtbarem, aufblasbarem, anergischem Kunststoff hergestellt ist und sich entlang einer geradlinigen Achse (X-X) zwischen einem ersten (29) und einem zweiten (21) abdichtbaren Ende erstreckt, um ein unter Druck stehendes Fluid aufzunehmen.

3. Phlebologische Vorrichtung (1) nach Anspruch 1 oder 2, wobei das röhrenförmige Element (2) aus mehreren einzelnen röhrenförmigen Elementen (2) gebildet ist, die an dem ersten (20) und dem zweiten Ende (21) miteinander derart verbunden sind, dass sie eine Kette (22) bilden.

4. Phlebologische Vorrichtung (1) nach einem der Ansprüche 1 bis 3, wobei die röhrenförmigen Elemente (2) durch Verbindungsmittel verbunden sind, die Klebstoff zum Verbinden zweier gegenüberliegender Innenstreifen des Schlauchs, ein Verschweißen der beiden gegenüberliegenden Innenstreifen des Schlauchs oder Klettverschlüsse, Druckknöpfe, Knöpfe und entsprechende Löcher, Haken und entsprechende Löcher umfassen.

5. Phlebologische Vorrichtung (1) nach einem der Ansprüche 2 bis 4, wobei die Haftschicht (3) ein Streifen aus biokompatiblem und anergischem Klebstoff ist, der sich entlang der Achse (X-X) des röhrenförmigen Elements (2) von seinem ersten (20) bis zu seinem zweiten (21) Ende und auf seinem mittleren Abschnitt erstreckt.

6. Phlebologische Vorrichtung (1) nach einem der Ansprüche 2 bis 4, wobei die Haftschicht (3) mindestens ein Streifen aus biokompatiblem und anergischem Klebstoff ist, der sich quer zu der Achse (X-X) bis zur Hälfte seiner Oberfläche erstreckt.

7. Phlebologische Vorrichtung (1) nach einem der Ansprüche 1 bis 6, wobei das röhrenförmige Element (2) mindestens ein Rückschlagventil für seine kontrollierte und einstellbare Befüllung umfasst.

8. Phlebologisches Kit, mit mindestens einem röhrenförmigen Element (2) gemäß Anspruch 1 aus aufblasbarem Kunststoff, das sich entlang einer Längsachse (X-X) erstreckt, einer Kompressionseinrichtung (5) für das röhrenförmige Element, wenn es aufgeblasen ist, eine Einrichtung zum Aufblasen des röhrenförmigen Elements und eine Einrichtung zum Abdichten des röhrenförmigen Elements.

9. Phlebologisches Kit nach Anspruch 8, wobei die Einrichtung zum Aufblasen eine manuelle oder elektrische Luftpumpe ist, die Einrichtung zum Abdichten eine Platte aufweist, die zum Verschmelzen zweier gegenüberliegender Streifen des röhrenförmigen Elements (2) aufheizbar ist.

## Revendications

1. - Dispositif phlébologique (1) comprenant au moins un élément tubulaire (2) apte à être mis sous pression et comprenant en outre des moyens de compression (5) dudit élément tubulaire applicables sur la surface externe du dispositif, **caractérisé par le fait que** ledit élément tubulaire (2) comporte au moins une partie externe revêtue d'une couche adhésive (3) pour un contact avec la peau d'un patient, ladite couche adhésive étant recouverte d'une bande protectrice amovible (4), et en outre, lesdits moyens de compression (5) pour ledit élément sont applicables sur la surface externe du dispositif, à l'opposé de ladite surface recouverte de la couche adhésive, et ils sont un bandage, une gaze, une bande élastique de compression ou des bas à porter autour de la partie de membre sur laquelle ledit élément tubulaire est appliqué.

2. - Dispositif phlébologique (1) selon la revendication 1, dans lequel ledit élément tubulaire (2) est fait de matière plastique anergique gonflable scellable et il s'étend le long d'un axe rectiligne (X-X) entre une première (29) et une seconde (21) extrémité scellable pour contenir un fluide sous pression.

3. - Dispositif phlébologique (1) selon la revendication 1 ou 2, dans lequel ledit élément tubulaire (2) est formé par une pluralité d'éléments tubulaires individuels (2) reliés entre eux à ladite première (20) et à ladite seconde (21) extrémité de façon à former une chaîne (22) .

4. - Dispositif phlébologique (1) selon l'une quelconque des revendications 1 à 3, dans lequel lesdits éléments tubulaires (2) sont reliés à l'aide de moyens de liaison comprenant de la colle pour assembler deux bandes internes opposées du tube, soudure desdites deux bandes internes opposées du tube, ou du Velcro, des boutons-pression, des boutons et des trous correspondants, des crochets et des trous correspondants.

5. - Dispositif phlébologique (1) selon l'une quelconque des revendications 2 à 4, dans lequel ladite couche adhésive (3) est une bande de colle biocompatible et anergique s'étendant le long dudit axe (X-X) de l'élément tubulaire (2) de sa première (20) à la seconde (21) extrémité et sur sa partie centrale.

6. - Dispositif phlébologique (1) selon l'une quelconque des revendications 2 à 4, dans lequel ladite couche adhésive (3) est au moins une bande de colle biocompatible et anergique s'étendant transversalement audit axe (X-X) jusqu'à la moitié de sa surface.

7. - Dispositif phlébologique (1) selon l'une quelconque des revendications 1 à 6, dans lequel ledit élément tubulaire (2) comprend au moins un clapet anti-retour pour son gonflage contrôlé et réglable.

8. - Kit phlébologique comprenant au moins un élément tubulaire (2) selon la revendication 1 fait de matière plastique gonflable s'étendant le long d'un axe longitudinal (X-X), des moyens de compression (5) dudit élément tubulaire lorsqu'il est gonflé, des moyens de gonflage dudit élément tubulaire, et un appareil de scellement dudit élément tubulaire.

9. - Kit phlébologique selon la revendication 8, dans lequel lesdits moyens de gonflage sont une pompe à air manuelle ou électrique, ledit appareil de scellement comprend une plaque qui peut être chauffée pour fusionner deux bandes opposées de l'élément tubulaire (2).
